# EUROPEAN PATENT APPLICATION

(11) **EP 3 108 931 A1**
(43) Date of publication of application: **28.12.2016**
(21) Application number: 15173639.4
(22) Date of filing: 24.06.2015
(51) Int. Cl.: A61N 5/06

(54) **COMBINATION OF AN ELECTRONIC DEVICE AND AN IRRADIATION DEVICE**

(71) Applicant: Medical IP B.V., 7571 BD Oldenzaal (NL)
(72) Inventor: Jámbor, Djuri Jozsef, 7577 KJ Oldenzaal (NL)
(74) Representative: 't Jong, Bastiaan Jacob

(57) **Abstract**

The invention relates to a combination, comprising:
- an electronic device, comprising a power supply, an external audio socket, electrically connected to the power supply, and controlling means for controlling the output of the power supply to the audio socket; and
- an irradiation device, comprising at least one laser for generating a laser beam, and an audio plug, electrically connected to the at least one laser, which audio plug is connected to the electronic device through the audio socket of the electronic device

wherein the orientation of the laser beam with respect to the electronic device is adjustable.

## Description

The invention relates to a combination, comprising:
- an electronic device, comprising a power supply, an external audio socket, electrically connected to the power supply, and controlling means for controlling the output of the power supply to the audio socket; and
- an irradiation device, comprising at least one laser for generating a laser beam, and an audio plug, electrically connected to the at least one laser, which audio plug is connected to the electronic device through the audio socket of the electronic device.

An example of such a combination known from the prior art comprises a laser device, electrically connected to an audio plug, which is connected to the electronic device through its audio socket. By such a connection, the laser device will be provided with power for generating a laser beam through the audio socket of the electronic device. This combination is usable as a laser pointer during slide show presentations. Since the electronic device is moreover portable, the combination may in such an application be oriented to direct the laser beam to a particular location, e.g. to bring a particular part of the slide show presentation to the attention of an audience.

Lasers may be used for a variety of applications, not limited to the use as a laser pointer. Lasers may for instance also be used for medical treatments. One example of such a medical treatment is the activation of the mitochondria in a cell such as a human cell by photochemical interaction between the laser and the mitochondria. Laser light may be absorbed by chromophores in the membranes of the mitochondria, thereby increasing cellular activity. It may as a consequence of the interaction increase ATP production and/or angiogenesis. It may as a consequence of the interaction induce proliferation of macrophages, lymphocytes, fibroblasts, endothelial cells and/or keratinocytes. It may as a consequence of the interaction stimulate the release of growth factors, prostaglandins, interleukins and other cytokines. It may as a consequence of the interaction also stimulate the synthesis of collagen. It may as a consequence of the interaction thus accelerate or assist in the treatment of inflammations and/or infections. It may also stimulate beta-endorphin and bradykinin, thereby influencing the pain threshold of the user. It may as a consequence of the interaction also reduce the effect of reactive oxygen species and thereby prevent apoptosis of epithelial and submucosal cells, which may be damaged by the use of chemotherapy or radiotherapy, which are typical methods for the treatment of cancer.

Such a treatment may for instance be applied by generating a laser beam which projects into the mouth cavity of a person. While the combination according to the prior art may be suitable as a laser pointer for slide show presentations, such a combination is cumbersome when used for medical treatments, since the laser beam is at a fixed position with respect to the electronic device, thereby requiring the user to frequently change the orientation of the combination in order to reach a large surface area of the mouth cavity. Since the electronic device is also mobile, it is required for the user to hold the device during a treatment. Moreover, the laser beam generated by the device according to the prior art has a wave length of approximately 620 nanometers. The absorption of laser light with such a wave length by mitochondria is however relatively limited. In addition, such a combination is not efficient in executing a treatment according to a prescription by a medical practitioner. It also does not allow verification of compliance by the user of the prescription.

It is now an object of the invention to provide a combination wherein the above stated drawbacks are reduced or even obviated.

This object is achieved with a combination according to the preamble, which is characterized in that the orientation of the laser beam with respect to the electronic device is adjustable.

With the combination according to the invention, it is possible to adjust the orientation of the laser beam without requiring a change in the orientation of the electronic device to which the irradiation device is connected. The freedom of orientation of the laser beam may be temporarily fixable in a reduced position, for instance by means on the device provided for that purpose.

The controlling means may be either software based or hardware based or a combination of both. An example of controlling means may be a software program loaded in a memory of the electronic device.

The controlling means may control the power supply to the audio socket such that the laser connected to the audio socket will either be in off, on or an intermittent or pulsating mode.

The audio plug is preferably a tip ring ring sleeve (TRRS) plug, which is suitable to extract a power of 7.5 mW and possibly even up to 15 mW from one conforming audio socket. The audio socket is external, e.g. arranged along the external surface of the electronic device. Such a plug will be able to provide sufficient power to the at least one laser when the power of the laser is tailored to that power.

Laser beam may also mean a bundle of one or more laser beams, in which the direction of the laser beams are parallel to each other. This increases the surface area reached by the laser beam.

The laser may be any suitable laser type, such as a laser diode or a laser fiber. Such a device, for instance a laser fiber, may be designed such that it is implementable in the human body, preferably by a medical practitioner.

With the combination according to the invention, it is possible to perform treatment sessions with the laser beam. Preferably, the combination of the laser, and its control via the controlling means are designed such that the absorbed energy is in a range between 2 and 4 Joules per square centimeter, to achieve adequate treatment.

In a preferred embodiment of the combination according to the invention, the combination is portable.

Portable in this regard means i.a. that the weight, size or shape of the combination is such that it is readily transportable by one person. This makes it easier to use the combination according to the invention at a variety of locations, and to carry the combination between these locations.

Portable may also mean that the power supply of the electronic device is wireless, i.e. is able to provide power without a connection to mains electricity. However, the power supply of the electronic device will generally be such that it is rechargeable by directly or indirectly connecting the device to mains electricity.

Non-limiting examples of electronic devices which are regarded portable are laptop computers, portable audio players such as MP3 players and mobile phones such as smartphones.

The irradiation device may be sold separately from the electronic device. Software based controlling means may also be sold or provided separately from the electronic device.

In another preferred embodiment of the combination according to the invention, the irradiation device comprises clamping means, for clamping the irradiation device to an edge of the mouth or the nose of a person.

For instance by providing a clamp adjacent to the laser, the irradiation device, and preferably the laser (or lasers) of the irradiation device, may be clamped to the edge or edges of the mouth and/or the nose of the person with the laser beam of the irradiation device facing the mouth cavity or inside the nostril respectively, to at least reduce the freedom of orientation of the laser beam with respect to the electronic device. The clamping means may take a form of two legs, connected to each other, each with a first end and a second end, wherein the first ends of the two legs are in contact with each other in a neutral position, in which position it is held by a spring force, and wherein the second ends of the two legs are distant from each other, and wherein the distance between the first ends is increased as the distance between the second ends is decreased. The clamping means are thus similar to a clothespin. The laser is preferably attached along one of the legs of the clamping means, preferably outside the contacting area of one leg with the other leg of the two legs.

Preferably, the clamping means are suitable for a clamping action in the corners of the mouth, which, due to their V-shaped form when the mouth is closed, provide a more firm attachment area compared to other parts of the edge of the mouth, thereby also maximally preventing damage of the clamping means to the lips of the user.

The clamping means may have a sensor for detecting proper attachment of the clamping means to the edge of a mouth or nose. A signal may be transmitted to the electronic device of such a situation. The controlling means of the device may be designed such that the irradiation device does not generate a laser beam until proper attachment.

In yet another preferred embodiment of the combination according to the invention, the irradiation device comprises a concave lens, arranged adjacent to the laser in the beam direction of the laser with a concave face substantially perpendicular to the beam direction of the laser.

It is preferred to provide a concave lens in the range of the laser beam in order to spread the beam from the laser across a larger area, thereby reducing the amount of lasers needed in order to increase the area reached by the device. Preferably, the optical axis of the lens is arranged substantially parallel to direction of the laser beam.

Preferably, the concave lens is plano-concave.

A plano-concave lens increases the area reached by the laser even further than a concave lens which is not plano-concave, e.g. which is concave on both sides of the lens in the beam direction.

In yet another preferred embodiment of the combination according to the invention, the wave length of the beam transmitted by the laser is (substantially) between 630 and 980 nanometers, more preferably (substantially) between 630 and 720 nanometers, most preferably substantially equal to 650 nanometers.

It has been shown that the absorption of the laser light by mitochondria is especially increased when the wave length of the beam transmitted by the laser is (or has a substantial component) between 630 and 980 nanometers or substantially within that range, more preferably between 630 and 720 nanometers or substantially within that range, and even more specifically substantially equal to 650 nanometers. Human blood cells and the human skin each have a different range of wavelengths optimal for absorption or laser light. While the absorption in both human blood cells and human skin is increased at a wavelength between 630 and 980 nanometers or substantially within that range, opposed to a wave length of 620 nanometers, the optimum of the combination of both curves is found in the more preferred range between 630 and 720 nanometers, more particularly around the even more preferred wavelength of 650 nanometers. In even another preferred embodiment of the combination according to the invention, the irradiation device comprises a flexible cable, connecting the laser of the irradiation device to the audio plug.

If the irradiation device comprises a flexible cable for connecting the laser to the audio plug, the freedom of movement of the laser with respect to the audio plug and, in combination, the electronic device, is increased. Similar cables are by itself known for the use in for instance headphones, charging cables, et cetera, but not in the combination according to the invention.

Preferably, the length of the cable between the laser (and, when applicable, preferably each laser) and the audio plug is longer than 120% of the length of the distance between the hip and the nose or mouth of an average person, in order to allow the irradiation device to stay clamped to the edge of the mouth or nose of a user while carrying the electronic device in the pocket of the user's trousers.

In again another preferred embodiment of the combination according to the invention, the irradiation device comprises at least two lasers, which at least two lasers are conjointly connected through the audio socket of the electronic device with one audio plug.

It is preferred if the irradiation device comprises at least two lasers, connected to one electronic device in a conjoint manner, i.e. where they both extract their power from a single audio plug which is connected to the audio socket of the electronic device. This increases the area reached by the irradiation device.

Preferably, the power of both or all of the at least two lasers is chosen to be equal to achieve an equal distribution of the intensity of the laser beams.

In another preferred embodiment of the combination according to the invention, the electronic device further comprises a user-controllable interface, wherein the controlling means are controllable through the interface.

While the controlling means may be self-controlled, they may also be comprise a user-controllable interface. Such a user-controllable interface may be presented on the screen of the electronic device. Preferably, the screen is a touchscreen to allow a more direct interaction between the user and the user-controllable interface of the controlling means. In a preferred embodiment, the electronic device is a smartphone (including devices without a capability of cellular connectivity which use the same operating system), and the controlling means comprise an application or app, installed in the memory of the electronic device.

The app may provide an overview of a treatment program, as prescribed by a medical practitioner and/or may allow the user to switch the laser between different modes and/or intensities.

The app may also save usage data in the memory of the electronic device in order to allow user compliance to be checked.

The combination according to the invention may comprise a software- and/or hardware-based security mechanism, allowing the irradiation device to only generate a laser beam when the app is installed on the electronic device, in order to prevent unintended or uncontrolled use of the device. The security mechanism may also or alternatively also prevent usage when the controlling means are not configured according to a treatment program, including a situation where a previous program has been completed.

In yet another a preferred embodiment of the combination according to the invention, the electronic device comprises alarming means, for reminding a person of the use of the irradiation device.

Alarming means, such as an alarm clock are preferred in order to remind a user to use the irradiation device. Preferably, the alarming means not only remind the user to start a treatment session of the treatment program, but also will remind the user if a treatment was aborted prematurely. Such alarming means preferably are implemented as a background process on the electronic device and preferably override a mute mode of the electronic device, preventing that the alarms given by the alarming means are missed or forgotten when the electronic device is for instance in hibernating or mute mode. The moments of time of alarms are preferably set by a medical practitioner. More preferably, these moments of time are preferably unchangeable by the user, or secured with a pass code, in order to increase treatment compliance.

In another preferred embodiment of the combination according to the invention, the controlling means are connected to connecting means, for connecting to an external server, for transmitting usage data of the irradiation device or a treatment program between the server and the electronic device.

The connecting means, which may be a data connection, preferably a wireless data connection, such as an internet connection available on the electronic device, allow the electronic device to communicate with an external server. Traffic between the electronic device and the external server is preferably coded to increase security and user privacy.

This may allow exchange of treatment programs (comprising one or more treatment sessions), e.g. the settings and schedule, from the external server to the electronic device. The user may be provided with a code, for example a numeric or alphanumeric code, which is entered in the app and submitted. The code may be transmitted to the external server, which returns to the electronic device with the treatment program assigned to that code. Since the prescribed treatment may depend on the user and the type of infection or inflammation, such an exchange is particularly preferred.

The connecting means may also allow the app to send information on the usage of the irradiation device to the external server, e.g. the number of treatment sessions completed, aborted or not executed. Thereby, user compliance may also be monitored externally from the electronic device. Preferably, such usage data is transmitted, preferably intermittently, as a background process in order to prevent manipulation by a user.

In again another preferred embodiment of the combination according to the invention, the electronic device comprises calling means.

The irradiation device according to the invention is preferably used in combination with a suitable electronic device which comprises calling means, such as a cellular phone connection. Many people already carry an electronic device with calling means, e.g. a mobile phone or smartphone, with them. It is therefore advantageous if the combination is carried out such that it is not necessary for a user to carry an additional electronic device suitable for driving the irradiation device.

In a further preferred embodiment of the combination according to the invention, the electronic device comprises call blocking means, for restraining the calling means when a laser beam is generated by the laser of the irradiation device.

Calling means may disturb or diminish the efficiency of a treatment by the irradiation device. It is therefore preferred if the electronic device comprises calling means which restrain, or preferably even block usage of the calling means when a treatment session is executed by the combination.

These and other features of the invention will be elucidated in conjunction with the accompanying figures.
Figure 1 shows a schematic overview of a combination according to the invention.
Figure 2 shows a part of the laser device according to the invention.

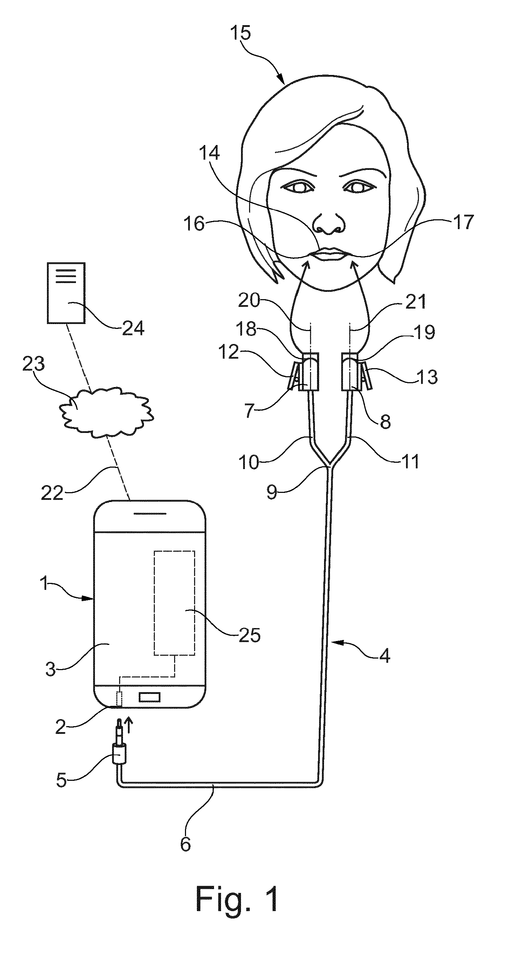

Figure 1 shows an electronic device 1, with an audio socket 2 facing the external surface of the device 1. The device 1 is a smartphone with a touch sensitive screen 3 for displaying a user interface on the screen 3 which can be controlled by touching the screen 3. An irradiation device 4 is connectable through insertion inside the audio socket 2 of the device 1 by its tip ring ring sleeve (TRRS) plug 5.

The irradiation device 4 further comprises a flexible cable 6, connecting the plug 5 to two lasers 7, 8. The cable 6 comprises a Y-joint 9 to split the cable 6 over a substantial part of the length of the cable 6 into subsegments 10, 11, allowing the lasers 7, 8 to be oriented and displaced with respect to the Y-joint 9 independently.

Each of the lasers 7, 8 comprises clamping means 12, 13, similar to a clothespin, with the laser attached to one of the legs of the clamping means 12, 13. The clamping means 12, 13 may be attached to the edge of the mouth 14 of a user 15, and preferably the V-shaped corners 16, 17, with the laser beam from the lasers 7, 8 projecting towards and inside the mouth cavity.

Each of the lasers 7, 8 also comprises a plano-concave lens 18, 19, which are arranged with their optical axes 20, 21 parallel to the laser beams of the respective lasers 7, 8.

The electronic device 1 is also connected through a mobile data connection 22 of the device with the internet 23, through which it is able to exchange treatment programs or usage data with an external server 24.

The electronic device 1 further comprises a rechargeable battery 25, for powering the audio socket, and via the plug 5 and cable 6, thereby powering lasers 7, 8.

Some details in figure 1 are drawn not on scale for clarification.

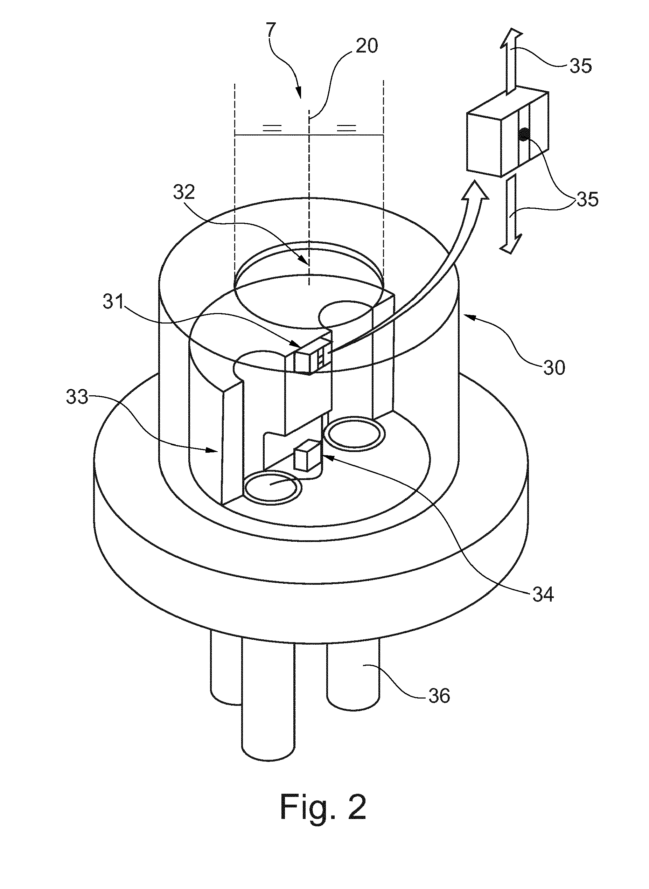

Figure 2 shows one of the lasers 7, 8 in an enlarged view. Details are shown for laser 7, but laser 8 is similar. The laser 7 comprises a protective can 30, with a laser diode 31 arranged inside the protective can 30. The laser diode 31 projects a laser beam through a window 32, in which the lens 18 is integrated. The laser 7 further comprises a heat sink 33 for cooling the laser diode 31 and a monitor photodiode 34, connected to the laser diode 31. The position of laser diode 31 may be adjustable along the beam direction 35. The ends 36 are electrically connected to the power supply 25 of the device 1 via cable 6, plug 5 and socket 2.

## Claims

1. Combination, comprising:
- an electronic device, comprising a power supply, an external audio socket, electrically connected to the power supply, and controlling means for controlling the output of the power supply to the audio socket; and
- an irradiation device, comprising at least one laser for generating a laser beam, and an audio plug, electrically connected to the at least one laser, which audio plug is connected to the electronic device through the audio socket of the electronic device
**characterized in that**
the orientation of the laser beam with respect to the electronic device is adjustable.

2. Combination according to claim 1, wherein the combination is portable.

3. Combination according to claim 1 or 2, wherein the irradiation device comprises clamping means, for clamping the irradiation device to an edge of the mouth or the nose of a person.

4. Combination according to claim 1, 2 or 3, wherein the irradiation device comprises a concave lens, arranged adjacent to the laser in the beam direction of the laser with a concave face substantially perpendicular to the beam direction of the laser.

5. Combination according to claim 4, wherein the concave lens is plano-concave.

6. Combination according to any of the preceding claims, wherein the wave length of the beam transmitted by the laser is (substantially) between 630 and 980 nanometers, more preferably (substantially) between 630 and 720 nanometers, most preferably substantially equal to 650 nanometers.

7. Combination according to any of the preceding claims, wherein the irradiation device comprises a flexible cable, connecting the laser of the irradiation device to the audio plug.

8. Combination according to any of the preceding claims, wherein the irradiation device comprises at least two lasers, which at least two lasers are conjointly connected through the audio socket of the electronic device with one audio plug.

9. Combination according to any of the preceding claims, wherein the electronic device further comprises a user-controllable interface, wherein the controlling means are controllable through the interface.

10. Combination according to any of the preceding claims, wherein the electronic device comprises alarming means, for reminding a person of the use of the irradiation device.

11. Combination according to any of the preceding claims, wherein the controlling means are connected to connecting means, for connecting to an external server, for transmitting usage data of the irradiation device or a treatment program between the server and the electronic device.

12. Combination according to any of the preceding claims, wherein the electronic device comprises calling means.

13. Combination according to claim 12, wherein the electronic device comprises call blocking means, for restraining the calling means when a laser beam is generated by the laser of the irradiation device.
